# EUROPÄISCHE PATENTANMELDUNG

(11) **EP 0 680 738 A1**
(43) Veröffentlichungstag der Anmeldung: **08.11.1995**
(21) Anmeldenummer: 95105980.7
(22) Anmeldetag: 21.04.1995
(51) Int. Cl.: A61F 11/14, G05B 19/408

(54) **Gehörschutzsystem an Produktionsmaschinen**

(30) Priorität: 04.05.1994 DE 4415666
(71) Anmelder: Hauni Maschinenbau Aktiengesellschaft, D-21033 Hamburg (DE)
(72) Erfinder: Heitmann, Uwe, D-21031 Hamburg (DE)

(57) **Zusammenfassung**

Es wird ein Gehörschutzsystem für das Personal an Maschinen beschrieben. Das System weist einen am Kopf zu tragenden Gehörschützer (6), einen in den Gehörschützer integrierten Empfangsteil (12) mit einem elektroakustischen Wandler zum Empfangen und Umwandeln drahtlos übermittelter Informationen in akustische Informationen und einen Sender (8) zum drahtlosen Übermitteln der Informationen zum Empfangsteil des Gehörschützers (6) auf.

Um die Akzeptanz des Gehörschützers durch das Personal zu erhöhen und die Betriebssicherheit zu verbessern ist der Sender (8) zum Übermitteln von vorgegebene Betriebszustände der Maschine (1) und/oder Produkteigenschaften repräsentierenden Zustandsinformationen in Form von Audiosignalen zum Empfangsteil (12) des Gehörschützers (6) an die Steueranordnung (4) der Maschine (1) angeschlossen. Außerdem ist ein Anschluß für ein Wiedergabegerät zum Abspielen von Tonträgern vorgesehen, so daß sich der Gehörschutzträger über den Sender (8) eine Geräuschkulisse nach eigenen Wünschen in den Gehörschützer einspielen kann, die im Bedarfsfall durch die akustischen Zustandsinformationen unterbrochen wird.

## Beschreibung

Die Erfindung betrifft ein Gehörschutzsystem für das Personal an Maschinen, insbesondere an Maschinen für die Herstellung, Verarbeitung und/oder Verpackung von stabförmigen Artikeln der tabakverarbeitenden Industrie, mit einem am Kopf zu tragenden Gehörschützer, einem dem Gehörschützer zugeordneten Empfangsteil mit wenigstens einem in den Gehörschützer integrierten elektroakustischen Wandler zum Empfangen und Umwandeln drahtlos übermittelter Informationen in akustische Informationen und einem Sender zum drahtlosen Übermitteln der Informationen zum Empfangsteil des Gehörschützers.

Beim Betrieb moderner Hochleistungsmaschinen ist die Entwicklung von Geräuschen und Lärm nicht zu vermeiden. Trotz aller erdenklichen Konstruktionsmaßnahmen zur Geräuschdämmung und -verhinderung übersteigt der Betriebslärm häufig die arbeitsschutzrechtlich zulässigen Obergrenzen, so daß zusätzliche Schutzmaßnahmen für das Bedienungspersonal erforderlich sind. Hierfür gibt es Gehörschutzeinrichtungen, die das Gehör des Operators oder anderer in der Nähe der Maschine beschäftigter Personen gegen hörbare Schallschwingungen abschirmen. Diese Gehörschützer werden wie Kopfhörer am Kopf getragen und weisen die Ohren bedeckende Gehörschutzkapseln auf. Die geräuschdämmende Wirkung der Gehörschützer hat allerdings den Nachteil, daß ihre Träger Umgebungsgeräusche geringerer Lautstärke nur noch sehr gedämpft oder gar nicht mehr wahrnehmen.

Um eine Verständigung mit dem Gehörschutzträger zu ermöglichen, ist es bekannt, in die Gehörschützer Einrichtungen zum Empfang von Informationen zu integrieren. Die DE-AS 27 01 963 beschreibt eine solche Gehörschutzeinrichtung mit einer Empfangseinrichtung und einem elektroakustischen Wandler zum Empfangen und akustischen Wiedergeben von Ultraschall-Signalen. Die Einrichtung dient zum Empfangen der natürlichen Ultraschall-Anteile der umgebenden Geräuschkulisse, deren Quelle dank der Peilwirkung des Empfangssystems auch räumlich geortet werden kann. Damit ist die Lokalisierung unerwünschter, eine Störung anzeigender Ultraschall-Maschinengeräusche möglich. Allerdings können auf diese Weise nur Fehler erkannt werden, die solche Ultraschallgeräusche im empfangenen Wellenlängenbereich verursachen. Eine darüber hinausgehende Kommunikation mit dem Gehörschutzträger ist nicht vorgesehen.

Eine andere Gehörschutzvorrichtung ist durch die DE-OS 29 25 134 bekanntgeworden. Diese Vorrichtung erzeugt aufgrund der in der Gehörschutzkapsel vorhandenen Geräusche einen phasenverdrehten Kompensationsschall, der den Störschall vermindert. Dabei ist auch die Einspeisung von zusätzlichen Informationen möglich, die über eine drahtlose Übertragungsstrecke, beispielsweise mit Infrarot- oder Ultraschallwellen erfolgen kann. Dies bezieht den Gehörschutzträger in ein vorhandenes Übertragungssystem des Betriebes zur Vermittlung von Nachrichten ein, so daß er trotz des Gehörschutzes nicht von der betrieblichen Kommunikation abgeschnitten ist. Die Übertragung von maschinenspezifischen Daten ist allerdings nicht vorgesehen.

Der vorliegenden Erfindung liegt die Aufgabe zugrunde, ein Gehörschutzsystem der eingangs beschriebenen Art weiter zu verbessern, insbesondere seine Brauchbarkeit und Akzeptanz zu erhöhen.

Gelöst wird diese Aufgabe erfindungsgemäß dadurch, daß der Sender zum Übermitteln von vorgegebene Betriebszustände der Maschine und/oder Produkteigenschaften repräsentierenden Zustandsinformationen in Form von Audiosignalen zum Empfangsteil des Gehörschützers an die Steueranordnung der Maschine angeschlossen ist. Auf diese Weise bleibt der Operator trotz der Gehörschutzmaßnahmen kommunikativ mit der Maschine verbunden, so daß er dort auftretende Fehler und sein Eingreifen erfordernde Betriebszustände unmittelbar akustisch wahrnehmen kann. Hierzu ist die Erfindung vorteilhafterweise dahingehend weitergebildet, daß die Steueranordnung mit Prüfeinrichtungen zum Erfassen vorgegebener Betriebszustände der Maschine und/oder Produkteigenschaften und zum Erzeugen entsprechender Zustandssignale verbunden ist und Mittel zum Verarbeiten der Zustandssignale zu Audiosignalen aufweist, welche drahtlos vom Sender zum Empfangsteil übertragbar und mittels des elektroakustischen Wandlers im Gehörschutz in akustische Zustandsinformationen konvertierbar sind. Zweckmäßigerweise werden nicht alle Prüfergebnisse der Prüfeinrichtungen zum Empfangsteil des Gehörschützers übertragen, um eine akustische Dauerberieselung des Operators zu vermeiden, sondern es werden für die erfaßten Maschinenzustände und Produkteigenschaften Grenzwerte gesetzt, deren Überschreiten eine akustische Meldung im Gehörschützer bewirkt.

Gemäß einer bevorzugten Weiterbildung der Erfindung ist vorgesehen, daß die Steueranordnung mit einer Spracherzeugungsanordnung verbunden ist, daß die Sprecherzeugungsanordnung Mittel zum Erzeugen von den Zustandssignalen entsprechenden Audiosignalen aufweist und daß die Audiosignale drahtlos vom Sender zum Empfangsteil übermittelbar und vom elektroakustischen Wandler im Gehörschützer in die erfaßten Maschinenzustände und/oder Produkteigenschaften repräsentierende akustische Sprachinformationen konvertierbar sind. Die Spracherzeugungsanordnung kann einen Sprachsyntheziser aufweisen, der in Abhängigkeit von den Zustandssignalen in vorgegebene akustische Sprachinformationen konvertierbare Audiosignale an den Sender abgibt. Eine andere Variante sieht vor, daß die Spracherzeugungsanordnung einen Speicher für in Form von Audiosignalsequenzen aufgezeichnete Sprachinformationen aufweist, daß sie Mittel zum Auswählen bestimmter Audiosignalsequenzen aus dem Speicher in Abhängigkeit von den Zustandssignalen und zum Übermitteln der ausgewählten Audiosignalsequenzen zum Sender aufweist. Auf diese Weise erhält der Operator akustische Fehlermeldungen im Klartext, die ihn befähigen, direkt an jeweils der richtigen Stelle einzugreifen. So ist eine schnellere Fehlerbehebung möglich. Außerdem ist die Übertragung von Sprachinformationen für die Bedienungsperson der Maschine angenehmer und akzeptabler als das Einspielen einfacher akustischer Warn- oder Alarmtöne.

Eine weitere Verbesserung der Akzeptanz des Gehörschutzsystems durch das Bedienungspersonal einer Maschine ergibt sich dadurch, daß mit dem Sender ein Abspielgerät zum Wiedergeben von auf Audio-Datenträgern gespeicherten Informationen verbunden ist. In das System können also handelsübliche Abspielgeräte für Tonbandcassetten oder Compactdisketten einbezogen werden, so daß sich der Maschinenbediener seine eigene Geräuschkulisse in den Gehörschützer einspielen kann. Eine weitere Ausgestaltung der Erfindung besteht darin, daß der Sender mit einem maschinenübergreifenden Kommunikationssystem verbunden ist. So ist das erfindungsgemäß vorgeschlagene Gehörschutzsystem auch in das allgemeine Kommunikationsnetz des Werkes eingebunden.

Damit nun jeweils aus den verschiedenen Informationsquellen die richtigen Informationen den Gehörschutzträger erreichen, sieht eine Weiterentwicklung der Erfindung vor, daß dem Sender eine mit allen Informationsquellen verbundene Auswahlanordnung vorgeschaltet ist, daß der Auswahlanordnung eine Rangfolge der Informationsquellen vorgegeben ist und daß die Auswahlanordnung bei Vorliegen von Informationen aus mehreren Informationsquellen jeweils die höherrangige Information auswählt und zum Sender überträgt. Durch diese Maßnahmen ist sichergestellt, daß der Gehörschutzträger über das allgemeine werksseitige Kommunikationssystem übertragene Nachrichten oder Fehlermeldungen von der Maschine auch dann erhält, wenn er über das Abspielgerät gerade Musik von eigenen Audio-Datenträgern hört. So sind sowohl die Sicherheit als auch die sachgerechte Betreuung der Maschine gewährleistet.

Als Sender kommt in erster Linie ein Infrarotsender und als Empfangsteil ein Infrarotempfänger in Frage. Da diese Einrichtungen nur einen räumlich begrenzten Wirkungsbereich haben, bleiben die mit dem Sender verbreiteten Informationen in der Nähe der betreffenden Maschine und beeinflussen nicht entsprechende Einrichtungen der Nachbarmaschinen. Um den Maschinenbereich lückenlos zu erreichen, können der Maschine mehrere unterschiedlich ausgerichtete Sender zugeordnet sein. Das System kann Mittel zum Begrenzen der Lautstärke der in die Gehörschutzkapseln abgegebenen akustischen Informationen aufweisen, um zu vermeiden, daß nun durch die Informationsübermittlung ihrerseits arbeitsrechtlich unzulässige Lautstärken im Gehörbereich erreicht werden.

Die Erfindung bietet den Vorteil eines Gehörschutzsystems hoher Akzeptanz dadurch, daß sich der Gehörschutzträger durch Auswahl von Tonträgern einen Geräuschhintergrund nach eigenen Wünschen schaffen kann. Dabei wird die Aufmerksamkeit des Gehörschutzträgers durch die Übermittlung spezieller maschinenbezogener Informationen auf die Ausführung bestimmter Bedienungsmaßnahmen gelenkt, sobald dies bei auftretenden Fehlern und Unregelmäßigkeiten erforderlich ist. Da die Übermittlung der maschinenbezogenen Informationen durch akustische Meldungen in Sprache unmittelbar zur Bedienungsperson erfolgt, ist das Eingreifen zur Fehlerbehebung nicht nur allein von der Wahrnehmung optischer Warnsignale abhängig. Die Fehlermeldung durch gesprochene Hinweise für den Operator hat den weiteren Vorteil, daß er unmittelbar erfährt, um welchen Fehler es sich jeweils handelt, so daß er unverzüglich dessen Behebung in Angriff nehmen kann. Die Fehlerbehebung wird also beschleunigt. Aus der geringen Reichweite der vorgesehenen drahtlosen Informationsübermittlung mit Infrarotwellenstrahlung ergibt sich der weitere Vorteil, daß sie benachbarte Maschinenbereiche nicht beeinflußt, also auf den Bewegungsbereich des Operators an dieser betreffenden Maschine beschränkt bleibt. Dieser kann erforderlichenfalls mit mehreren Sendern lückenlos versorgt werden. Von der Nachrichtenübertragung des Betriebes ist der Operator mit dem erfindungsgemäßen System nicht abgeschnitten, weil dieses Gehörschutzsystem mit dem Kommunikationssystem des Betriebes verbunden werden kann.

Die Erfindung wird nun anhand der Zeichnung näher erläutert.

Es zeigen
- Figur 1: eine schematische Darstellung des erfindungsgemäß vorgeschlagenen Gehörschutzsystems an einer Produktionsmaschine der tabakverarbeitenden Industrie und
- Figur 2: eine Blockdarstellung der Systemkomponenten.

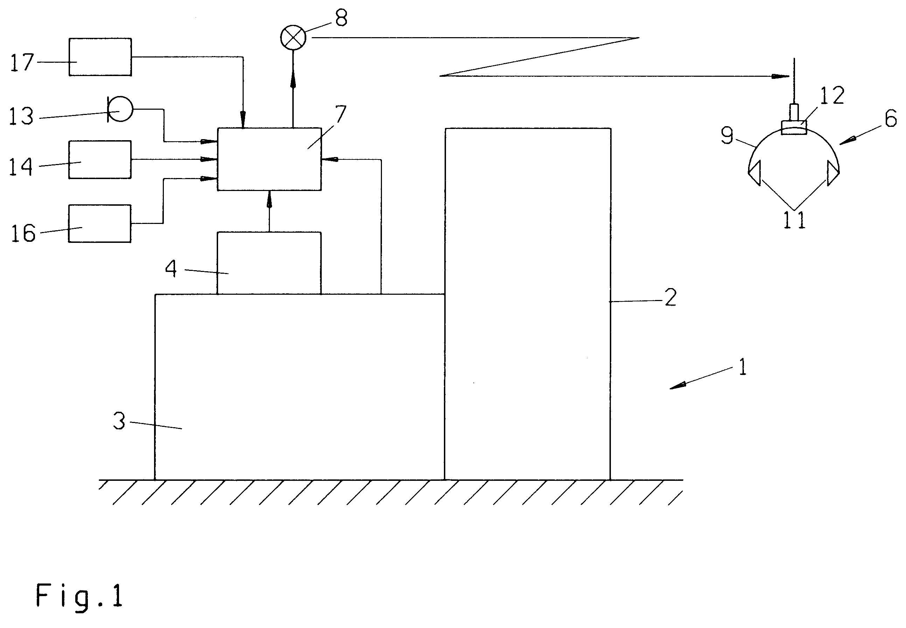

In Figur 1 ist als Ausführungsbeispiel schematisch eine Maschine 1 der tabakverarbeitenden Industrie mit einem Gehörschutzsystem gemäß der Erfindung dargestellt. Die Maschine 1 ist beispielsweise eine Zigarettenstrangmaschine des Typs Protos der Anmelderin, die aus einer Verteilereinheit 2 und einer Strangeinheit 3 besteht und eine Steueranordnung 4 des Typs SRM aufweist. Derartige Maschinen sind bekannt und bedürfen hier daher keiner weiteren Beschreibung.

Moderne Zigarettenstrangmaschinen dieser Art laufen mit Produktionsgeschwindigkeiten von 7.000 bis hinauf zu 16.000 Zigaretten pro Minute. Eine Lärmentwicklung, die auch über die arbeitsschutzrechtlich festgelegten Höchstgrenzen hinausgeht, ist trotz des Einsatzes aller Konstruktions- und bautechnischen-Maßnahmen nicht zu vermeiden. In einer Produktionshalle ist in der Regel eine große Anzahl solcher Maschinen installiert, deren Geräuschemissionen sich addieren und für die in der Halle anwesenden Personen gesundheitsschädliche Werte annehmen können. Die bisher praktikabelste Schutzmaßnahme ist der individuelle Schallschutz durch Abkapselung des Gehörs mittels am Kopf zu tragender Gehörschützer. Um diesen Gehörschutz für die betroffenen Personen akzeptabler zu machen, die Personen in die betriebliche Kommunikation einzubeziehen und eine sachgerechte Bedienung und Wartung der Maschine sicherzustellen, ist das Gehörschutzsystem gemäß der Erfindung vorgesehen.

Dieses gemäß der Erfindung der Maschine 1 zugeordnetes Gehörschutzsystem weist einen am Kopf zu tragenden Gehörschützer 6 und eine mit der Steueranordnung 4 der Maschine verbundene Sendeanordnung 7 mit einem Sender 8 auf.

Der Gehörschützer besteht aus einem Kopfbügel 9, zwei Gehörkapseln 11 und einem Empfangsteil 12. Die Gehörkapseln 11 sind schalldämmend ausgebildet und schirmen das Gehör gegen den Maschinenlärm ab. Vom Sender 8 ausgestrahlte Audioinformationen werden vom Empfangsteil 12 empfangen und von in den Gehörkapseln 11 angeordneten, in der Zeichnung nicht dargestellten elektroakustischen Wandlern in akustische Informationen umgewandelt, die der Gehörschutzträger in den Gehörkapseln 11 wahrnimmt.

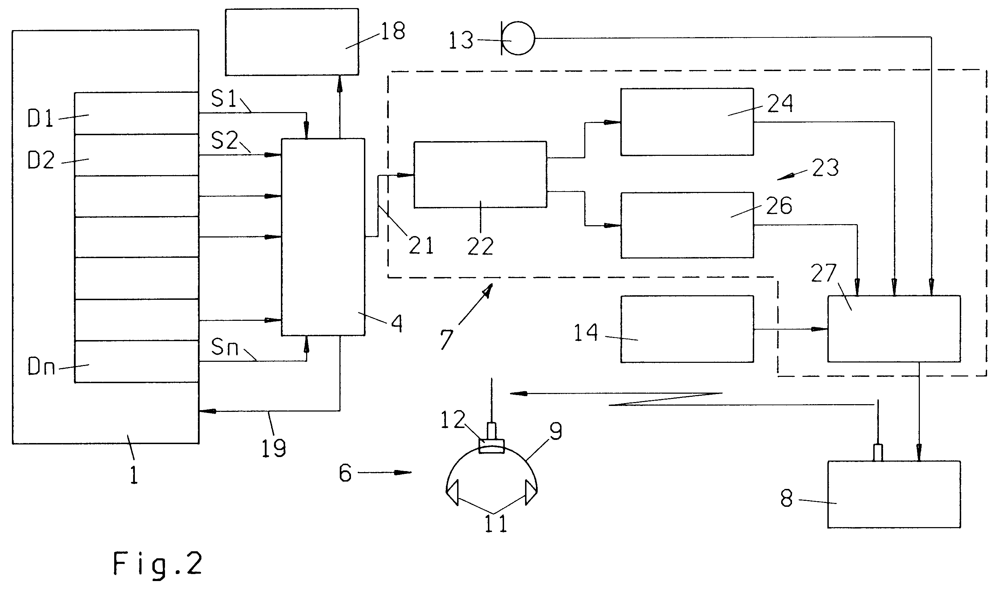

Die mit dem Sender 8 verbundene Sendeanordnung 7 ist an die Steueranordnung 4 der Maschine angeschlossen. Auf diese Weise können von der Steueranordnung 4 der Maschine erzeugte Zustandssignale in niederfrequente Audiosignale verwandelt und über den Sender 8 drahtlos zum Empfangsteil 12 des Gehörschützers 6 übermittelt werden. Hierzu folgen weiter unten im Zusammenhang mit der Figur 2 weitere Erläuterungen.

Mit 13 ist ein Mikrofon bezeichnet, welches die Verbindung der Sendeanordnung 7 mit einer werkseigenen Kommunikationseinrichtung symbolisiert. Über diese Verbindung können Durchsagen der Werks- oder Hallenleitung zum Operator übermittelt werden.

An die Sendeanordnung 7 ist außerdem ein Laufwerk 14 anschließbar, mit welchem Tonträger wie Tonbandcassetten oder Musik-Compactdisketten (CD) abgespielt werden können. Dieses Laufwerk kann je nach Wunsch vom Operator angeschlossen werden oder auch in die Maschine integriert sein. Auf diese Weise wird es dem Gehörschutzträger ermöglicht, in den Gehörschutzkapseln eine Geräuschkulisse nach seinen eigenen Wünschen zu erzeugen. Mit 16 ist eine werkseigene Einrichtung zum Übermitteln eines Unterhaltungsprogramms bezeichnet, die ebenfalls an die Sendeanordnung 7 angeschlossen sein kann. Damit können vom Werk, in dem die Maschine 1 installiert ist, bereitgestellte Programme zum Gehörschutzträger übermittelt werden. Mit 17 ist eine Einrichtung zum Einstellen und Begrenzen des Pegels der Sendeleistung bezeichnet, welche sicherstellt, daß der durch die Informationsübertragung am Ohr erzeugte Schalldruck zulässige Höchstgrenzen nicht überschreitet.

In der Figur 2 ist das Gehörschutzsystem gemäß der Erfindung in einem Blockdiagramm dargestellt, in welchem die einzelnen Blöcke die Funktionen des Systems versinnbildlichen.

Die Maschine 1 weist Prüf- bzw. Meßeinrichtungen D1 bis Dn auf, mit denen verschiedene Betriebszustände der Maschine und Produkteigenschaften geprüft bzw. gemessen werden. In einer Zigarettenstrangmaschine können dies u.a. Einrichtungen zum Erfassen der Tabakfeuchte im Stauschacht, der Überschußmenge im Tabakstrang, der Strangdichte, von Softspots in dem Zigarettenstreng, des Strangdurchmessers usw. sein. Auch Meß- und Prüfeinrichtungen sich anschliessender Maschinen, beispielsweise einer Filteransetzmaschine, die mit der Strangmaschine eine Maschinenkombination bildet, können hier angeschlossen sein und verwertet werden. Die Prüf- und Meßeinrichtungen D1 bis Dn geben den Eigenschaften der Produkte bzw. den Betriebszuständen der Maschine entsprechende Prüfsignale S1 bis Sn, die im vorliegenden Zusammenhang auch als Zustandssignale bezeichnet werden, an die Steueranordnung 4 ab, welche die verschiedenen Aggregate der Maschine 1 entsprechend regelt oder steuert, was durch eine Verbindung 19 symbolisiert ist. Dieser übliche Steuer- und Regelvorgang ist nicht Gegenstand der vorliegenden Erfindung und braucht daher nicht näher beschrieben zu werden. Auf einem mit der Steueranordnung 4 verbundenen Monitor 18 können Betriebszustände der Maschine und Produkteigenschaften abgelesen werden.

Über- oder unterschreitet eines der Zustandssignale S1 bis Sn, einen vorgegebenen Grenzwert, weil das zugehörige Produkt- oder Betriebskriterium unakzeptable Werte annimmt, so erzeugt die Steueranordnung 4 aus dem betreffenden Zustandssignal ein Stör- oder Fehlersignal 21 und gibt dieses außer an den Monitor 18 und ggf. weitere Alarmeinrichtungen auch an die als Mittel zum Verarbeiten der Zustandssignale zu Audiosignalen vorgesehene Sendeanordnung 7 ab. Dort gelangt das Fehlersignal 21 in eine Ablaufsteuerung 22, welche es identifiziert und ihm einen bestimmten Code zuordnet. Das codierte Fehlersignal gelangt in eine Spracherzeugungsanordnung 23, welche es in Audiosignale umwandelt. Als Spracherzeugungsanordnung 23 kann ein Sprachsynthesizer 24 vorgesehen sein, der dem Fehlersignal entsprechende Audiosignale generiert. Als Spracherzeugungsanordnung kann auch eine Prozessoranordnung 26 mit einem Audiospeicher dienen, in dem den Fehlersignalen entsprechende Audiosignalsequenzen gespeichert sind. Die Audiosignalsequenzen sind Meldungstexte oder Bruchstücke von Meldungstexten, die auf Tonband, Compactdiskette (CD) oder in Speicherchips eines dem Prozessor 26 zugeordneten elektronischen Massenspeichersgespeichert sind. In Abhängigkeit von einem Zustands- oder Fehlersignal greift der Prozessor 26 auf das betreffende Speichermedium zu und ruft die Audiosignalsequenzen ab, die er für die Darstellung des aufgetretenen Fehlers als Audiosignal benötigt. Sind die Fehlermeldungen komplett als Audiosignalsequenzen gespeichert, so wird der Prozessor in Abhängigkeit von einem eintreffenden Fehlersignal 21 die zugehörige Audiosignalsequenz aus dem Audiospeicher auswählen und sie zum Sender 8 übermitteln, der sie zum Empfangsteil 12 überträgt, wo sie der elektroakustische Wandler in eine gesprochene Information für den Gehörschutzträger verwandelt.

Das Speichern kompletter Fehlermeldungen als Audiosignalsequenzen erfordert relativ viel Speicherplatz. Werden dagegen Bruchstücke von Fehlermeldungen als Audiosignalsequenzen gespeichert, so brauchen in verschiedenen Meldungen vorkommende übereinstimmende Bruchstücke im Speicher jeweils nur einmal als Audiosignalsequenzen bereitgehalten zu werden. Das verringert den Speicherbedarf. Der Prozessor 22 wählt in Abhängigkeit von einem Fehlersignal 21 jeweils die für die Erzeugung der zugehörigen Meldung benötigten Audiosignalsequenzen aus, fügt sie zur Meldung zusammen und übermittelt diese, wie oben beschrieben, zum Sender 8, der sie zum Empfangsteil 12 des Gehörschützers überträgt.

Die Ablaufsteuerung 22 und der Prozessor 26 sowie ggf. eine als Massenspeicher ausgebildete elektronische Speicheranordnung können in einem herkömmlichen Personalcomputer (PC) mit entsprechender Soundkarte und Audiosoftware realisiert sein. Diese Form der Meldungsaufbereitung hat den Vorteil, daß die erzeugten Audiosignale zu akustischen Informationen mit menschlicher Stimme führen und daher einen natürlichen Klang haben. Die von dem Sprachsynthesizer 24 abgegebenen Audiosignale führen dagegen zu einer sog. Computerstimme, die eher maschinell klingt.

Die von der Spracherzeugungsanordnung 23 abgegebenen Audiosignale gelangen zu einer Niederfrequenz-Steuereinheit 27 (NF-Steuereinheit) und von dort zum Sender 8, welcher sie drahtlos zum Empfangsteil 12 des Gehörschützers 6 übermittelt. Der Sender 8 kann ein Infrarotsender sein, dessen geringe Reichweite gewährleistet, daß die gesendeten Informationen nicht in den Bereich anderer Maschinen gelangen und dort Störungen verursachen können. Reicht ein Sender für die Versorgung eines Arbeitsraumes einer Maschine nicht aus, so können in verschiedenen Positionen mehrere Sender angeordnet sein, welche den gesamten Maschinenbereich abdecken.

Wie bereits im Zusammenhang mit der Figur 1 beschrieben, ist die Sendeanordnung 7 mit der Kommunikationseinrichtung des Werkes verbunden, was durch das Mikrofon 13 symbolisiert ist. Diese Kommunikationseinrichtung ist ebenfalls mit der NF-Steuereinheit 27 verbunden. Außerdem ist an die NF-Steuereinheit 27 ein Wiedergabegerät 14 für Tonträger, beispielsweise ein Cassettenabspielgerät oder ein CD-Player angeschlossen.

Im Normalbetrieb ermöglichen die von den Prüfeinrichtungen D1 bis Dn abgegebenen Zustandssignale S1 bis Sn eine automatische Steuerung bzw. Regelung der verschiedenen Funktionen der Maschine 1, die das Eingreifen der Bedienungsperson nicht erfordert. Solange die Maschine 1 normal und fehlerfrei läuft, kann der Träger des Gehörschützers 6 sich durch Einlegen einer Musikcassette oder einer Musik-CD in das Laufwerk 14 in den Gehörkapseln 11 eine Geräuschkulisse nach eigenen Wünschen erzeugen. Tritt ein Fehler in der Maschine 1 auf, der sich dadurch zeigt, daß wenigstens eines der Zustandssignale S1 bis Sn einen Grenzwert überschreitet, so erzeugt die Steueranordnung 4 ein Fehlersignal 21, welches im Sprachsynthesizer 24 oder im Prozessor 26 in der oben beschriebenen Art und Weise ein Audiosignal hervorruft, welches über die NF-Steuereinheit 27 die Musikübertragung aus dem Laufwerk 14 unterbricht und über den Sender 8 selbst zum Empfangteil 12 des Gehörschützers 6 übertragen wird. Der Träger des Gehörschützers 6 wird also unmittelbar von dem Fehler in der Maschine informiert. Ist als Spracherzeugungsanordnung ein Synthesizer 24 vorgesehen, welcher aus maschinell erzeugten Lauten gesprochene Buchstaben und Wörter bildet, so klingt die erzeugte akustische Meldung in den Gehörschutzkapseln 11 eher monoton und unnatürlich. Wird stattdessen der Prozessor 26 mit einer Audio-Speicheranordnung eingesetzt, in welcher aus natürlicher Sprache gebildete Audiosignalsequenzen aufgezeichnet sind und entsprechend den Fehlermeldungen abgerufen werden, so wird die Wiedergabe durch die elektroakustischen Wandler in den Gehörschutzkapseln 11 des Gehörschützers 6 den Klang einer natürlichen Stimme haben.

Die NF-Steuereinheit 27 gibt eine gewünschte Priorität der verschiedenen Audio-Signalquellen vor. Dabei wird in der Regel das Laufwerk 14 die geringste Priorität haben und von Audiosignalen aus der Spracherzeugungsanordnung 23 ausgeblendet werden. Der Kommunikationsanordnung, die durch das Mikrofon 13 repräsentiert ist, kann je nach Bedarf eine noch höhere oder die mittlere Priorität eingeräumt werden.

Die Spracherzeugungseinheit 23 kann sowohl einen Sprachsynthesizer 24 als auch eine Prozessoreinheit 26 mit Audiospeicher aufweisen. In diesem Fall können bestimmte Meldungen vom Sprachsynthesizer erzeugt, andere vom Prozessor 26 aus dem Audiospeicher abgerufen werden. In der Regel wird man sich aber mit einem der Spracherzeugungsmittel in der Spracherzeugungseinheit 23 begnügen.

## Patentansprüche

1. Gehörschutzsystem für das Personal an Maschinen, insbesondere an Maschinen für die Herstellung, Verarbeitung und/oder Verpackung von stabförmigen Artikeln der tabakverarbeitenden Industrie, mit einem am Kopf zu tragenden Gehörschützer, einem dem Gehörschützer zugeordneten Empfangsteil mit wenigstens einem in den Gehörschützer integrierten elektroakustischen Wandler zum Empfangen und Umwandeln drahtlos übermittelter Informationen in akustische Informationen und einem Sender zum drahtlosen Übermitteln der Informationen zum Empfangsteil des Gehörschützers, dadurch gekennzeichnet, daß der Sender (8) zum Übermitteln von vorgegebene Betriebszustände der Maschine (1) und/oder Produkteigenschaften repräsentierenden Zustandsinformationen in Form von Audiosignalen zum Empfangsteil (12) des Gehörschützers (6) an die Steueranordnung (4) der Maschine (1) angeschlossen ist.

2. System nach Anspruch 1, dadurch gekennzeichnet, daß die Steueranordnung (4) mit Prüfeinrichtungen (D1 bis Dn) zum Erfassen vorgegebener Betriebszustände der Maschine (1) und/oder Produkteigenschaften und zum Erzeugen entsprechender Zustandssignale (S1 bis Sn) und mit einer Sendeanordnung (7) mit Mitteln (7) zum Verarbeiten der Zustandssignale zu Audiosignalen verbunden ist, welche drahtlos vom Sender (8) zum Empfangsteil (12) übertragbar und mittels des elektroakustischen Wandlers im Gehörschutz (6) in akustische Zustandsinformationen konvertierbar sind.

3. System nach Anspruch 1 oder 2, dadurch gekennzeichnet, daß die Steueranordnung (4) mit einer Spracherzeugungsanordnung (23) verbunden ist, daß die Spracherzeugungsanordnung Mittel (24,26) zum Erzeugen von den Zustandssignalen entsprechenden Audiosignalen aufweist und daß die Audiosignale drahtlos vom Sender (8) zum Empfangsteil (12) übermittelbar und vom elektroakustischen Wandler im Gehörschützer (6) in die erfaßten Maschinenzustände und/oder Produkteigenschaften repräsentierende akustische Sprachinformationen konvertierbar sind.

4. System nach Anspruch 3, dadurch gekennzeichnet, daß die Spracherzeugungsanordnung (23) einen Sprachsynthesizer (24) aufweist, der in Abhängigkeit von den Zustandssignalen in vorgegebene akustische Sprachinformationen konvertierbare Audiosignale an den Sender (8) abgibt.

5. System nach einem oder mehreren der vorangehenden Ansprüche, dadurch gekennzeichnet, daß die Spracherzeugungsanordnung (23) einen Prozessor (26) und einen Speicher für in Form von Audiosignalsequenzen aufgezeichnete Sprachinformationen aufweist und daß der Prozessor zum Auswählen bestimmter Audiosignalsequenzen aus dem Speicher in Abhängigkeit von den Zustandssignalen und zum Übermitteln der ausgewählten Audiosignalsequenzen zum Sender (8) ausgebildet ist.

6. System nach einem oder mehreren der vorangehenden Ansprüche, dadurch gekennzeichnet, daß mit dem Sender (8) ein Abspielgerät (14) zum Wiedergeben von auf Audio-Datenträgern gespeicherten Informationen verbunden ist.

7. System nach einem oder mehreren der vorangehenden Ansprüche, dadurch gekennzeichnet, daß der Sender (8) mit einem maschinenübergreifenden Kommunikationssystem (13) verbunden ist.

8. System nach einem oder mehreren der vorangehenden Ansprüche, dadurch gekennzeichnet, daß dem Sender (8) eine mit allen Informationsquellen verbundene NF-Steuereinheit (27) vorgeschaltet ist, daß der NF-Steuereinheit (27) eine Rangfolge der Informationsquellen vorgegeben ist und daß die NF-Steuereinheit bei Vorliegen von Informationen aus mehreren Informationsquellen jeweils die höherrangige Information auswählt und zum Sender (8) überträgt.

9. System nach einem oder mehreren der vorangehenden Ansprüche, dadurch gekennzeichnet, daß als Sender (8) ein Infrarotsender und als Empfangsteil (12) ein Infrarotempfänger vorgesehen sind.

10. System nach einem oder mehreren der vorangehenden Ansprüche, dadurch gekennzeichnet, daß der Maschine (1) mehrere räumlich unterschiedlich ausgerichtete Sender (8) zugeordnet sind.

11. System nach einem oder mehreren der vorangehenden Ansprüche, dadurch gekennzeichnet, daß Mittel (17) zum Begrenzen der Lautstärke der abgegebenen akustischen Informationen vorgesehen sind.
